# EUROPEAN PATENT SPECIFICATION

(11) **EP 1 532 981 B1**
(45) Date of publication and mention of the grant of the patent: **26.09.2007**
(21) Application number: 03795230.6
(22) Date of filing: 22.08.2003
(51) Int. Cl.: A61K 31/5575, A61K 9/08, A61K 47/16, A61P 27/06

(54) **STABLE EYE DROPS CONTAINING LATANOPROST AS THE ACTIVE INGREDIENT**
STABILE AUGENTROPFEN ENTHALTEND LATANOPROST ALS WIRKSTOFF
GOUTTES OPHTALMIQUES STABLES CONTENANT COMME PRINCIPE ACTIF DU LATANOPROST

(30) Priority: 23.08.2002 JP 2002243955; 20.11.2002 JP 2002336242
(43) Date of publication of application: 25.05.2005
(73) Proprietor: SANTEN PHARMACEUTICAL CO., LTD., Higashiyodogawa-ku, Osaka-shi, Osaka 533-8651 (JP)
(72) Inventor: ASADA, Hiroyuki, c/o SANTEN PHARMACEUTICAL CO. LTD, Osaka-shi, Osaka 533-8651 (JP); KIMURA, Akio, c/o SANTEN PHARMACEUTICAL CO. LTD, Osaka-shi, Osaka 533-8651 (JP)
(74) Representative: Peaucelle, Chantal
(86) International application number: PCT/JP2003/010607
(87) International publication number: WO 2004/024164

(56) References cited:
- EP-A- 0 850 926
- WO-A1-00/03736
- JP-A- 2001 081 048
- JP-A- 2001 114 700
- JP-A- 2002 161 037
- US-A- 6 011 062

## Description

### Technical Field

The present invention provides a latanoprost ophthalmic solution which can be stored at room temperature.

### Background Art

Latanoprost is a prostaglandin-type therapeutic agent for glaucoma represented by a chemical name of isopropyl (Z)-7[(1R,2R,3R,5S)3,5-dihydroxy-2-[(3R)-3-hydroxy-5-phenylpentyl]cyclopentyl]-5-heptanoate. Latanoprost is a selective FP receptor agonist and lowers intraocular pressure by promoting outflow of an aqueous humor (Japanese Patent No. 2721414). An administration route of latanoprost is instillation, and an ophthalmic solution containing 0.005% latanoprost (trade name: Xalatan ophthalmic solution) is commercially available (hereinafter referred to as "commercially available ophthalmic solution"). As stated in the attached statement of the commercially available ophthalmic solution, its pH is adjusted to 6.7, and it contains benzalkonium chloride, sodium chloride, sodium dihydrogenphosphate monohydrate and anhydrous disodium hydrogenphosphate as additives.

However, since the commercially available ophthalmic solution lacks stability, it is necessary to store it in a cold environment (2° to 8°C) shielding the light.

There is a paper which reports stability of the commercially available ophthalmic solution to a temperature and light (Journal of Glaucoma, 10 (5), 401-405, 2001). However, there has been no report concerning means of stabilizing an ophthalmic solution containing latanoprost.

EP 0 850 926 relates to difluoroprotaglandin derivatives and their use to prevent or heat an eye disease. A composition comprising latanaprost at a concentration of 0.01% (WN) is disclosed.

US 6 011 062 discloses stable pharmaceutical compositions comprising protaglandins but does not give results with latanaprost.

WO 00 03736 discloses an aqueous solution containing prostaglandin derivatives.

JP 2002 161037, JP 2001 114700, and JP 2001 081048 disclose the use of ε-aminocaproic acid as a buffering agent or as a stabilizing and pH adjusting agent, but do not teach means for stabilizing latanaprost.

### Disclosure of the Invention

Thus, since it is inconvenient to handle the commercially available ophthalmic solution in storing it as described above, it has been desired to develop a latanoprost ophthalmic solution which can be stored at room temperature and is excellent in stability.

The present inventors first focused attention on the fact that pH of the commercially available ophthalmic solution is adjusted to 6.7 and studied precisely effects of pH on stability of latanoprost. As a result, the present inventors found that when pH becomes too alkaline or too acidic, stability of latanoprost lowers, and when pH is adjusted in a specific range of 5.0 to 6.25, latanoprost is stabilized to give a latanoprost ophthalmic solution which can be stored at room temperature.

The inventors also focused attention on additives and studied precisely effects of various additives on stability of latanoprost. As a result, the present inventors found that when ε-aminocaproic acid is added, latanoprost is stabilized to give a latanoprost ophthalmic solution which can be stored at room temperature.

Namely, the present invention provides an ophthalmic solution comprising latanoprost as an active ingredient, wherein latanoprost is stabilized to be stored at room temperature by at least one means selected from the following 1) and 2);
1) adjusting pH of the solution to 5.0 to 6.25 and
2) adding ε-aminocaproic acid to the solution.

A concentration of latanoprost, which is the active ingredient of the ophthalmic solution in the present invention, is preferably 0.001 to 0.005% (W/V), particularly preferably 0.005% (W/V).

One of the characteristics of the present ophthalmic solution is that pH of the solution is adjusted to 5.0 to 6.25 to stabilize latanoprost. The pH range is acceptable as pH of ophthalmic solutions. As details are described in stability tests in Examples, stability of latanoprost was found to be greatly affected by a change in pH.

A pH adjusting agent can be used in order to adjust pH to 5.0 to 6.25. Examples of pH adjusting agents are hydrochloric acid, citric acid, phosphoric acid, acetic acid, sodium hydroxide, potassium hydroxide, sodium carbonate, sodium hydrogencarbonate and the like.

On the other hand, latanoprost can be stabilized by adding ε-aminocaproic acid to the solution other than by adjusting pH. A concentration of ε-aminocaproic acid, depending on a concentration of latanoprost, is usually 0.1 to 2% (W/V), preferably 0.2 to 1% (W/V). It was also found that when the method wherein ε-aminocaproic acid is added is used, stability is kept at pH closer to approximate neutrality, namely at pH of about 7.0, too.

Though various additives are used in order to stabilize ophthalmic solutions, ε-aminocaproic acid exhibits an excellent effect on stabilization of latanoprost among many additives as apparent from the section of stability tests.

Of course, pH can be adjusted to 5.0 to 6.25 and ε-aminocaproic acid can be added as the additive at the same time, and thereby their synergistic effect can be obtained.

An additive such as a buffer, a tonicity agent, a solubilizer, a preservative or a viscous agent can be optionally added other than the above-mentioned pH adjusting agent and ε-aminocaproic acid in order to prepare the ophthalmic solution of the present invention.

Examples of buffers are phosphoric acid or salts thereof, boric acid or salts thereof, citric acid or salts thereof, acetic acid or salts thereof, tartaric acid or salts thereof, trometamol and the like.

Examples of tonicity agents are glycerin, propylene glycol, sodium chloride, potassium chloride, sorbitol, mannitol and the like.

Examples of solubilizers are polysorbate 80, polyoxyethylene hydrogenated castor oil, macrogol 4000 and the like.

Examples of preservatives are benzalkonium chloride, benzethonium chloride, sorbic acid, potassium sorbate, methyl p-hydroxybenzoate, propyl p-hydroxybenzoate, chlorobutanol and the like.

Examples of viscous agents are hydroxypropylmethylcellulose, hydroxypropylcellulose, polyvinyl alcohol, carboxyvinyl polymers, polyvinylpyrrolidone and the like.

Latanoprost was stabilized by adjusting pH of the ophthalmic solution comprising latanoprost as the active ingredient in the range of 5.0 to 6.25, and thereby it is possible to provide the latanoprost ophthalmic solution which can be stored at room temperature and is excellent in stability.

Latanoprost was also stabilized by adding ε-aminocaproic acid to an aqueous latanoprost solution, and thereby it is possible to provide the latanoprost ophthalmic solution which can be stored at room temperature and is excellent in stability.

### Brief Description of Drawings

Fig. 1 is a graph showing changes of residual ratios of latanoprost with time at each pH value when a latanoprost ophthalmic solution was stored at 60°C.
Fig. 2 is a graph showing changes of residual ratios of latanoprost with time at each pH value when a latanoprost ophthalmic solution was stored at 70°C
Fig. 3 is a graph showing changes of residual ratios of latanoprost with time when a test solution obtained by adding each additive to a latanoprost solution was stored at 50°C.
Fig. 4 is a graph showing changes of residual ratios of latanoprost with time when a test solution obtained by adding each additive to a latanoprost solution was stored at 80°C.

### Best Mode for Carrying out the Invention

Examples of the present invention are shown below. All ophthalmic solutions prepared in Examples exhibit excellent stability at room temperature.

### Example 1

Crystalline sodium dihydrogenphosphate (1 g) was dissolved in purified water (ca. 80 ml), a 1 N aqueous sodium hydroxide solution was added thereto to adjust pH to 5.0, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost. After the temperature of the solution was returned to room temperature, pH was confirmed to be 5.0.

### Example 2

Crystalline sodium dihydrogenphosphate (1 g) was dissolved in purified water (ca. 80 ml), a 1 N aqueous sodium hydroxide solution was added thereto to adjust pH to 5.5, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost. After the temperature of the solution was returned to room temperature, pH was confirmed to be 5.5.

### Example 3

Crystalline sodium dihydrogenphosphate (1 g) was dissolved in purified water (ca. 80 ml), a 1 N aqueous sodium hydroxide solution was added thereto to adjust pH to 6.0, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost. After the temperature of the solution was returned to room temperature, pH was confirmed to be 6.0.

### Example 4

Crystalline sodium dihydrogenphosphate (1 g) was dissolved in purified water (ca. 80 ml), a 1 N aqueous sodium hydroxide solution was added thereto to adjust pH to 6.25, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost. After the temperature of the solution was returned to room temperature, pH was confirmed to be 6.25.

### Example 5

Crystalline sodium dihydrogenphosphate (1 g), sodium chloride (0.4 g) and benzalkonium chloride (0.02 g) were dissolved in purified water (ca. 80 ml), a 1 N aqueous sodium hydroxide solution was added thereto to adjust pH to 6.0, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost. After the temperature of the solution was returned to room temperature, pH was confirmed to be 6.0.

Next, stability of the latanoprost ophthalmic solution at different pH was studied.

### [Stability test of latanoprost 1]

### Experimental method

1) Latanoprost (0.0025 g) was weighed out in a 50 ml-beaker, a phosphate buffer (50 ml) having each pH (4.0, 5.0, 5.5, 6.0, 6.25, 6.5, 6.7 or 8.0) prepared in advance was added to the beaker, and the mixture was stirred with a magnetic stirrer. The mixture was stirred while warming it in a water bath at about 80°C for about 30 minutes to dissolve latanoprost.
2) It was confirmed that latanoprost was dissolved, and pH was confirmed.
3) A glass ampoule was charged with each prepared solution (2.5 ml) and sealed by melting it.
4) It was stored at 60°C or 70°C.
5) Sampling was carried out with time until 28th day after starting storage, latanoprost contents were measured by high performance liquid chromatography, and residual ratios were calculated. Samples having residual ratios of 95% or higher after storage at 60°C for 28 days and residual ratios of 90% or higher after storage at 70°C for 28 days were judged to be stable.

### Results

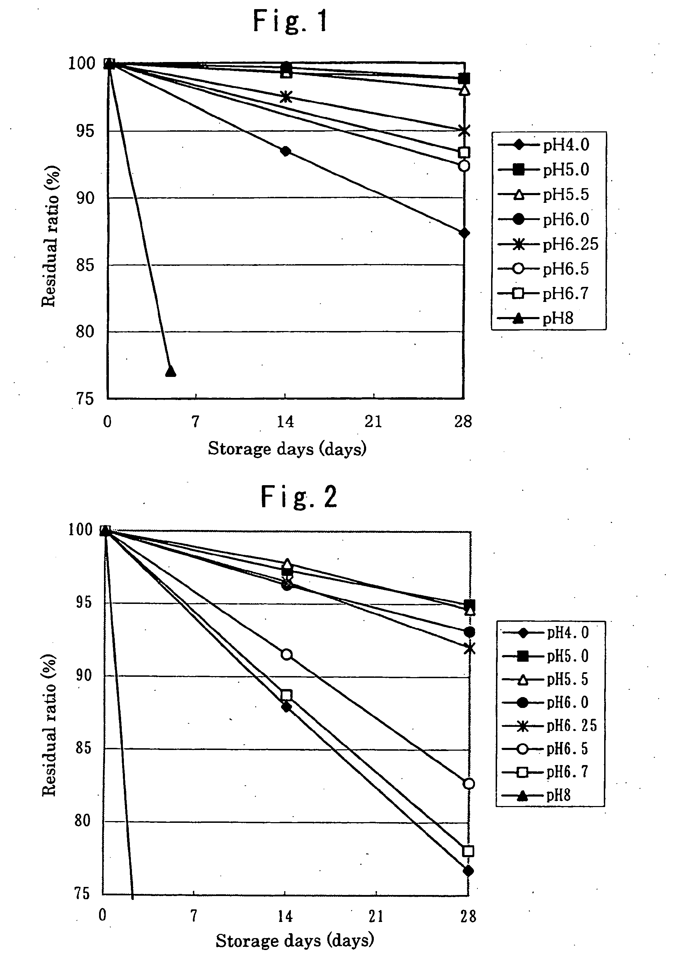

Changes of residual ratios with time during storage at 60°C and 70°C are shown in Figs. 1 and 2 respectively. Residual ratios after storage for 28 days are shown in Table 1. As apparent from Table 1, in the case of storage at 60°C, residual ratios of 95% or higher, namely stable samples, were in the range of pH of 5.0 to 6.25. Similarly, in the case of storage at 70°C, residual ratios of 90% or higher, namely stable samples, were also in the range of pH of 5.0 to 6.25.

From the above-mentioned results, it was found that when pH of the latanoprost ophthalmic solution is adjusted to 5.0 to 6.25, latanoprost is stabilized, and the ophthalmic solution can be stored at room temperature.

The residual ratio of latanoprost after storage at 70°C for 28 days was lower than 80% at pH of 6.7, though pH of 6.7 is the same value as that of the commercially available ophthalmic solution.

**Table 1**

| Stability of latanoprost (Residual ratio (%) after storage for 28 days) | | | | | | | | |
|---|---|---|---|---|---|---|---|---|
| | pH 4.0 | pH 5.0 | pH 5.5 | pH 6.0 | pH 6.25 | pH 6.5 | pH 6.7 | pH 8.0 |
| 60°C | 87.4 | 98.9 | 98.0 | 98.9 | 95.0 | 92.4 | 93:4 | 30.0* |
| 70°C | 76.7 | 94.9 | 94.6 | 93.1 | 92.0 | 82.7 | 78.1 | 14.1** |

| | | | | | | | | |
|---|---|---|---|---|---|---|---|---|
| *Value on 21st day, ** value on 12th day | | | | | | | | |

### Example 6

ε-Aminocaproic acid (1 g), concentrated glycerin (1.8 g) and benzalkonium chloride (0.01 g) were dissolved in purified water (ca. 80 ml), pH was adjusted to 6.7, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost in the vehicle. After the temperature of the obtained solution was returned to room temperature, pH was confirmed to be 6.7.

### Example 7

ε-Aminocaproic acid (0.2 g), concentrated glycerin (2.3 g) and benzalkonium chloride (0.01 g) were dissolved in purified water (ca. 80 ml), pH was adjusted to 6.7, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost in the vehicle. After the temperature of the obtained solution was returned to room temperature, pH was confirmed to be 6.7.

### Example 8

ε-Aminocaproic acid (1 g), concentrated glycerin (1.8 g) and benzalkonium chloride (0.01 g) were dissolved in purified water (ca. 80 ml), pH was adjusted to 6.0, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost in the vehicle. After the temperature of the obtained solution was returned to room temperature, pH was confirmed to be 6.0.

### Example 9

ε-Aminocaproic acid (1 g), concentrated glycerin (1.8 g) and benzalkonium chloride (0.01 g) were dissolved in purified water (ca. 80 ml), pH was adjusted to 7.0, and purified water was added to the mixture so that total volume was 100 ml to give a vehicle. The vehicle (100 ml) was added to latanoprost (5 mg), and the mixture was stirred while warming it in a water bath at about 80°C to dissolve latanoprost in the vehicle. After the temperature of the obtained solution was returned to room temperature, pH was confirmed to be 7.0.

### [Stability test of latanoprost 2]

Effects of various additives on stability of latanoprost were studied. Crystalline sodium dihydrogenphosphate, polyethylene glycol 400 (PEG 400), polyethylene glycol, trehalose, isopropanol, α-cyclodextrin, sodium citrate and ε-aminocaproic acid were used as additives. Crystalline sodium dihydrogenphosphate was added in formulation of additives having no buffer capacity in order to avoid an effect due to a change in pH.

### Experimental method

Each additive was dissolved in purified water (ca. 80 ml) so that its concentration was each value in Table 2, pH was adjusted to 7.0, and purified water was added to the solution so that total volume was 100 ml to give each vehicle. Each vehicle (100 ml) was added to latanoprost (5 mg), the mixture was stirred while warming it in a water bath at about 80°C. After the temperature of the obtained solution was returned to room temperature, pH was confirmed to be 7.0. The obtained solution was used as a test solution. A glass ampoule was charged with each test solution (approximately 2.5 ml) and stored in an incubator at 50°C or 80°C. After a prescribed period, the test solution was sampled, each latanoprost content was determined by high performance liquid chromatography, and each residual ratio to each content before storage was determined.

**Table 2**

| | Formulation 1 | Formulation 2 | Formulation 3 | Formulation 4 | Formulation 5 | Formulation 6 | Formulation 7 | Formulation 8 |
|---|---|---|---|---|---|---|---|---|
| Latanoprost | 0.005% | 0.005% | 0.005% | 0.005% | 0.005% | 0.005% | 0.005% | 0.005% |
| Crystalline sodium dihydrogenphosphate | 1% | 1% | 1% | 1% | 1% | 1% | - | - |
| PEG 400 | - | 1% | - | - | - | - | - | - |
| Propylene glycol | - | - | 1% | - | - | - | - | - |
| Trehalose | - | - | - | 1% | - | - | - | - |
| Isopropanol | - | - | - | - | 1% | - | - | - |
| α-Cyclodextrin | - | - | - | - | - | 0.11% | - | - |
| Sodium citrate | - | - | - | - | - | - | 1% | - |
| ε-Aminocaproic acid | - | - | - | - | - | - | - | 1% |
| Diluted hydrochloric acid | q.s. | q.s. | q.s. | q.s. | q.s. | q.s. | q.s. | q.s. |
| Sodium hydroxide | q.s. | q.s. | q.s. | q.s. | q.s. | q.s. | q.s. | q.s. |
| Purified water | q.s. | q.s. | q.s. | q.s. | q.s. | q.s. | q.s. | q.s. |
| pH | 7.0 | 7.0 | 7.0 | 7.0 | 7.0 | 7.0 | 7.0 | 7.0 |

| | | | | | | | | |
|---|---|---|---|---|---|---|---|---|
| q.s.: quantum sufficit | | | | | | | | |

### Results

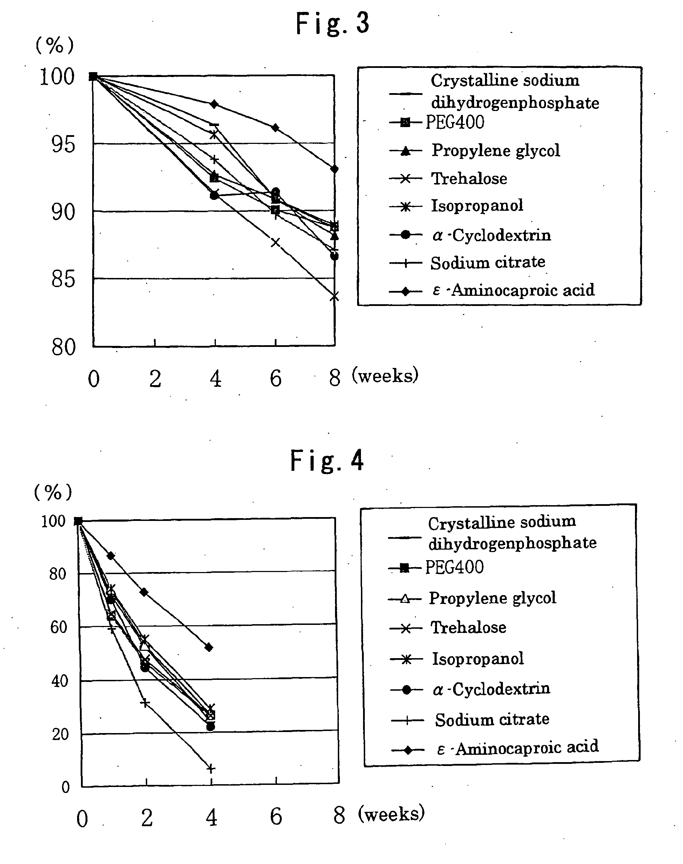

Changes in residual ratio with time during storage at 50°C and 80°C are shown in Figs. 3 and 4 respectively. Residual ratios after storage at 50°C for eight weeks and at 80°C for four weeks are shown in Table 3. As apparent from Table 3, in the case of storage at 50°C, the residual ratio in formulation wherein ε-aminocaproic acid was added was 90% or higher, and the stabilization effect of ε-aminocaproic acid is higher than those of the other additives. Table 3 shows that in the case of storage at 80°C, while residual ratios in other formulations were 30% or lower, the residual ratio in the formulation wherein ε-aminocaproic acid was added was 51.8%, and the stabilization effect of ε-aminocaproic acid is high as well as the case of storage at 50°C.

The above-mentioned results show that when ε-aminocaproic acid is added to latanoprost, latanoprost is stabilized and can be stored at room temperature.

**Table 3**

| | Additives | Storage at 50°C. for eight weeks | Storage at 80°C for four weeks |
|---|---|---|---|
| Formulation 1 | Crystalline sodium dihydrogenphosphate | 88.7% | 24.0% |
| Formulation 2 | PEG 400 | 88.8% | 25.9% |
| Formulation 3 | Propylene glycol | 88.1% | 26.1% |
| Formulation 4 | Trehalose | 83.7% | 26.4% |
| Formulation 5 | Isopropanol | 88.9% | 28.9% |
| Formulation 6 | α-Cyclodextrin | 86.6% | 22.1% |
| Formulation 7 | Citric acid | 87.1% | 6.3% |
| Formulation 8 | ε-Aminocaproic acid | 93.1% | 51.8% |

### Industrial Applicability

The present invention provides a latanoprost ophthalmic solution which can be stored at room temperature and is excellent in stability.

## Claims

1. An ophthalmic solution comprising latanoprost at a concentration of 0.001-0.005% (W/V) as an active ingredient, wherein latanoprost is stabilized to be stored at room temperature by at least one means selected from the following 1) and 2);
1) adjusting pH of the solution to 5.0 to 6.25 and
2) adding ε-aminocaproic acid to the solution.

2. The ophthalmic solution as claimed in claim 1, wherein a concentration of latanoprost is 0.005% (W/V).

3. The ophthalmic solution as claimed in claim 1 or 2, wherein the concentration of ε-aminocaproic acid is 0.1 to 2% (W/V).

4. The ophthalmic solution as claimed in claim 3, wherein the concentration of ε-aminocaproic acid is 1% (W/V).

5. A method of stabilizing an ophthalmic solution comprising latanoprost at a concentration of 0.001-0.005% (W/V) as an active ingredient, comprising stabilizing latanoprost contained in the ophthalmic solution to store latanoprost at room temperature by at least one means selected from the following 1) and 2);
1) adjusting pH of the solution to 5.0 to 6.25 and
2) adding ε-aminocaproic acid to the solution.

## Patentansprüche

1. Ophthalmische Lösung, die Latanoprost mit einer Konzentration von 0,001 - 0,005 % (w/v) als Wirkstoff umfasst, wobei Latanoprost durch wenigstens eines der aus den folgenden 1) und 2) ausgewählten Mittel so stabilisiert ist, dass es bei Raumtemperatur gelagert werden kann:
1) Einstellung des pH-Wertes der Lösung auf 5,0 bis 6,25 und
2) Zugabe von ε-Aminocapronsäure zur Lösung.

2. Ophthalmische Lösung nach Anspruch 1, wobei die Konzentration von Latanoprost 0,005 % (w/v) beträgt.

3. Ophthalmische Lösung nach Anspruch 1 oder 2, wobei die Konzentration der ε-Aminocapronsäure 0,1 bis 2 % (w/v) beträgt.

4. Ophthalmische Lösung nach Anspruch 3, wobei die Konzentration der ε-Aminocapronsäure 1 % (w/v) beträgt.

5. Verfahren zur Stabilisierung einer ophthalmischen Lösung, die Latanoprost mit einer Konzentration von 0,001 - 0,005 % (w/v) als Wirkstoff umfasst, umfassend die Stabilisierung des in der ophthalmischen Lösung enthaltenen Latanoprosts durch wenigstens eines der aus den folgenden 1) und 2) ausgewählten Mittel so, dass es bei Raumtemperatur gelagert werden kann:
1) Einstellung des pH-Wertes der Lösung auf 5,0 bis 6,25 und
2) Zugabe von ε-Aminocapronsäure zur Lösung.

## Revendications

1. Solution ophtalmique comprenant du latanoprost à une concentration de 0,001% à 0,005% (p/v) en tant que principe actif, dans laquelle le latanoprost est stabilisé pour être stocké à température ambiante par au moins un moyen choisi parmi 1) et 2) ;
1) l'ajustement du pH de la solution entre 5,0 et 6,25 et
2) l'ajout d'acide ε-aminocaproïque à la solution.

2. Solution ophtalmique selon la revendication 1, dans laquelle la concentration de latanoprost est de 0,005% (p/v).

3. Solution ophtalmique selon la revendication 1 ou 2, dans laquelle la concentration d'acide ε-aminocaproique est comprise entre 0,1% à 2% (p/v).

4. Solution ophtalmique selon la revendication 3, dans laquelle la concentration d'acide ε-aminocaproïque est de 1% (p/v).

5. Procédé de stabilisation d'une solution ophtalmique comprenant du latanoprost à une concentration comprise entre 0,001% à 0,005% (p/v) en tant que principe actif, comprenant du latanoprost stabilisé présent dans la solution ophtalmique pour stocker le latanoprost à température ambiante par au moins un moyen choisi parmi 1) et 2) ;
1) l'ajustement du pH de la solution entre 5,0 et 6,25 et
2) l'ajout d'acide ε-aminocaproïque à la solution.
